# FASCICULE DE BREVET EUROPEEN

(11) **EP 1 165 035 B1**
(45) Date de publication et mention de la délivrance du brevet: **26.10.2005**
(21) Numéro de dépôt: 00915224.0
(22) Date de dépôt: 27.03.2000
(51) Int. Cl.: A61K 7/48

(54) **COMPOSITIONS COSMETIQUES CONTENANT AU MOINS UNE SUBSTANCE DESTINEE A AUGMENTER LA FONCTIONNALITE ET/OU L'EXPRESSION DES RECEPTEURS MEMBRANAIRES CD44 DES CELLULES DE LA PEAU**
KOSMETISCHE ZUSAMMENSETZUNGEN DIE MINDESTENS EINE SUBSTANZ ZUR VERBESSERUNG DER FUNKTIONSFÄHIGKEIT UND/ODER ZUR ERHÖHUNG DER EXPRESSION DES MEMBRANREZEPTORS CD44 IN HAUTZELLEN ENTHALTEN
COSMETIC COMPOSITIONS CONTAINING AT LEAST ONE SUBSTANCE FOR INCREASING THE FUNCTIONALITY AND/OR EXPRESSION OF CD44 MEMBRANE RECEPTORS OF SKIN CELLS

(30) Priorité: 26.03.1999 FR 9903840
(43) Date de publication de la demande: 02.01.2002
(73) Titulaire: PARFUMS CHRISTIAN DIOR, 75008 Paris (FR)
(72) Inventeur: DUMAS, Marc, 45100 Orléans (FR); BONTE, Frédéric, 45100 Orléans (FR)
(74) Mandataire: Giraud, Françoise
(86) Numéro de dépôt international: PCT/FR2000/000764
(87) Numéro de publication internationale: WO 2000/057836

(56) Documents cités:
- EP-A- 0 756 866
- EP-A- 0 770 399
- WO-A-00/13661
- WO-A-00/27353
- WO-A-96/19228
- WO-A-98/17320
- D.F. WILLIAMS: "CHEMISTRY AND TECHNOLOGY OF THE COSMETICS AND TOILETRIES INDUSTRY" 1996 , BLACKIE ACADEMIC & PROFESSIONAL XP002129103 page 128-130

## Description

L'invention concerne des compositions cosmétiques contenant au moins une substance destinée à augmenter la fonctionnalité et/ou l'expression des récepteurs membranaires CD44 des cellules de la peau.

L'acide hyaluronique est présent en quantité abondante dans l'épiderme humain où il se trouve localisé dans les espaces intercellulaires situés entre les kératinocytes.

L'acide hyaluronique est également présent en quantité abondante dans le derme humain où il se trouve localisé dans les espaces intercellulaires situés entre les fibroblastes.

La glycoprotéine appelée CD44 est présente dans l'épiderme à la surface des kératinocytes et sa fonction est de permettre la liaison de l'acide hyaluronique à la surface des kératinocytes.

La glycoprotéine appelée CD44 est également présente dans le derme à la surface des fibroblastes et sa fonction est de permettre la fixation de l'acide hyaluronique, des collagènes, notamment des collagènes I et IV, ainsi que de la fibronectine à la surface de ces cellules.

L'acide hyaluronique est le capteur hydrique naturel de l'épiderme et du derme puisqu'il est capable de retenir jusqu'à mille fois son volume en eau. On voit donc bien ici l'importance pour la cellule de posséder des récepteurs capables de fixer cet hydrocapteur afin de retenir l'eau à son contact et permettre son hydratation.

Il est connu, par ailleurs, que les dépôts d'acide hyaluronique coïncident avec une activité cellulaire de renouvellement et de migration intense dans différents tissus et organes en développement. Dans l'épiderme, il est localisé dans les parties vivantes profondes, au niveau basal et suprabasal, là où le renouvellement cellulaire et la migration sont les plus importants. Cependant, aux endroits où l'adhérence cellulaire au constituant matriciel est essentielle comme au niveau de l'interface entre les cellules basales épidermiques et la jonction dermo-épidermique, le CD44 est absent.

Un autre élément important pour la physiologie de l'épiderme et du derme est que, en situation inflammatoire, les régions envahies par les cellules inflammatoires, lymphocytes et neutrophiles, présentent une disparition en CD44 et en acide hyaluronique. Or cette disparition est nécessaire à une adhérence forte des cellules inflammatoires et à leur invasion et leur installation dans les tissus (CD44 Substituted with Heparan Sulfate an Endo-β-galactosidase-Sensitive Oligosaccharides : A Major Proteoglycan in Adult Human Epidermis. J. Invest. Dermatol. ; 1997 ; 109 ; pp 213-218).

Il est par ailleurs connu que l'acide hyaluronique permet d'établir de véritables ponts entre deux récepteurs CD44 portés par des cellules adjacentes. Ceci est étayé par le fait que, lorsque l'on traite une peau maintenue en survie par de la hyaluronidase qui dégrade l'acide hyaluronique, on ouvre les espaces intercellulaires et on provoque une acanthose, c'est-à-dire une hyperplasie épidermique. Une telle acanthose avec disparition de CD44 est aussi observée au niveau des sites épidermiques envahis par les leucocytes.

Toutes ces données prouvent que le CD44 a un rôle important dans le développement, la maintenance et la fonctionnalité de l'épiderme.

Les inventeurs ont, de plus, constaté une diminution des liaisons entres les récepteurs CD44 et l'acide hyaluronique avec l'âge. Ce phénomène est à l'origine de l'augmentation de la sécheresse de l'épiderme avec l'âge, ainsi que de la diminution du renouvellement des cellules de l'épiderme. En effet avec l'âge, l'épiderme s'atrophie et se renouvelle moins bien.

De toutes ces connaissances, il ressort que tout moyen permettant d'augmenter la fonctionnalité des récepteurs transmembranaires CD44 et d'améliorer ainsi la fixation de l'acide hyaluronique à la surface de la cellule, constitue un moyen intéressant pour améliorer l'hydratation au niveau cellulaire de l'épiderme et du derme.

Par ailleurs, tout moyen permettant d'intensifier le réseau d'interconnexion entre les cellules de l'épiderme par fixation d'acide hyaluronique sur des récepteurs CD44 de deux cellules adjacentes constitue un moyen particulièrement efficace de limiter la migration des cellules inflammatoires telles que les lymphocytes et les neutrophiles vers les cellules de l'épiderme. Une telle action aura pour effet de prévenir ou traiter les phénomènes d'inflammation.

Enfin, il ressort que tout moyen permettant d'augmenter l'expression et/ou la fonctionnalité des récepteurs transmembranaires CD44 et d'améliorer ainsi la fixation de l'acide hyaluronique à la surface de la cellule, constitue un moyen intéressant pour lutter contre ou, au moins, ralentir et/ou retarder l'apparition des signes du vieillissement cutané, tels que notamment les rides, la sécheresse cutanée, l'altération du teint, l'altération des propriétés biomécaniques de la peau tels que le relâchement, la perte de tonicité, de fermeté et d'élasticité de la peau.

Les effets des acides alpha-hydroxylés sur le dévelopement dermique, la synthèse de collagène, la synthèse de l'acide hyaluronique, le contenu de ADN et l'expression de protéines de l'épiderme ont été rapportés par D.F. Williams dans "Chemistry and Technology of the Cosmetics and Toiletries Industry (1996).

La présente invention résulte de la mise en évidence après une étude systématique de ce que certaines substances agissent de façon efficace sur la fonctionnalité et sur l'expression des récepteurs CD44 des cellules de l'épiderme. Cette découverte a conduit les inventeurs de la présente invention à introduire les substances dont l'activité avait ainsi été mise en évidence dans des compositions cosmétiques ou dermatologiques, notamment destinées au maintien ou à la restauration de l'hydratation au niveau cellulaire de l'épiderme, et à ralentir et/ou retarder l'apparition des signes du vieillissement cutané.

Ainsi donc, l'invention résulte, pour l'essentiel, de la découverte de ce qu'il était possible d'agir par l'intermédiaire d'agents actifs cosmétiques ou pharmaceutiques à usage topique sur l'expression et/ou la fonctionnalité des récepteurs membranaires CD44 des cellules de la peau, ce qui a permis d'envisager l'utilisation de ces agents actifs dans des procédés de traitement cosmétique ou thérapeutique à usage topique de la peau.

Par "augmentation de l'expression" on entend qu'il se produit une augmentation de la quantité de récepteurs CD44 fabriqués par les cellules.

Par "augmentation de la fonctionnalité des récepteurs CD44" on entend au sens de l'invention qu'il se produit une amélioration de la fixation, à la surface des cellules concernées, de l'acide hyaluronique et/ou du collagène, en particulier du collagène I et/ou du collagène IV, et/ou de la fibronectine.

L'invention concerne donc des utilisations dans le domaine cosmétique mettant en oeuvre cette découverte. L'homme du métier choisira bien sûr des actifs acceptables dans le domaine visé.

Ainsi, l'invention concerne l'utilisation non thérapeutique dans une composition cosmétique, du gluconate de calcium ou d'un sel de l'acide gluconique associé à un sel de calcium, en tant qu'agent actif améliorant l'hydratation de l'épiderme et/ou du derme ou améliorant la fixation à la surface des cellules de la peau de l'acide hyaluronique, et/ou du collagène, en particulier du collagène I et/ou du collagène IV, et/ou de la fibronectine.

Plus précisément, selon ce premier aspect, l'invention concerne l'utilisation non thérapeutique, dans une composition cosmétique, du gluconate de calcium ou d'un sel de l'acide gluconique associé à un sel de calcium, en tant qu'agent actif augmentant l'expression des récepteurs membranaires CD44 des cellules de la peau et/ou améliorant la fixation à la surface desdites cellules de la peau de l'acide hyaluronique, et/ou du collagène, en particulier du collagène I et/ou du collagène IV, et/ou de la fibronectine.

Selon une première variante, l'utilisation de cet agent actif permet d'améliorer la fonctionnalité des récepteurs membranaires CD44 des cellules de la peau en ce qu'ils fixent à la surface desdites cellules, l'acide hyaluronique et/ou le collagène, en particulier le collagène I et/ou le collagène IV, et/ou la fibronectine.

Il a été constaté que cette action sur les récepteurs membranaires CD44 était ressentie aussi bien sur les récepteurs CD44 des kératinocytes que des fibroblastes.

Ainsi, selon une première variante, ledit agent actif augmente l'expression des récepteurs CD44 des kératinocytes et/ou des fibroblastes et/ou améliore la fixation de l'acide hyaluronique à la surface de ces cellules.

Selon une autre variante, ledit agent actif augmente l'expression des récepteurs CD44 des fibroblastes et/ou améliore la fixation à la surface desdits fibroblastes du collagène, en particulier du collagène I et/ou du collagène IV, et/ou de la fibronectine.

Comme exposé précédemment, l'invention résulte d'une étude systématique d'un grand nombre d'agents actifs. Cette étude a permis de montrer, en particulier, que les agents actifs les plus efficaces sur l'expression et/ou la fonctionnalité des récepteurs membranaires CD44 des cellules de la peau s'avéraient être le gluconate de calcium et les sels d'acide gluconique associé à un sel de calcium.

Les sels d'acide gluconique sont avantageusement choisis parmi les sels de sodium, de calcium, de magnésium, de zinc et de manganèse.

Enfin, comme cela ressort des exemples, il est apparu que le gluconate de calcium présentait un intérêt tout particulier, de même que l'acide gluconique associé à un sel de calcium, par exemple à du chlorure de calcium.

Il ressort également des exemples que, dans le cas où l'on utilise un gluconate en présence de calcium, le rapport molaire du calcium au gluconate est avantageusement compris entre 2 et 6, de préférence de l'ordre de 4.

Les proportions d'agents actifs dans les compositions cosmétiques de l'invention peuvent varier dans de larges gammes. Toutefois, il est avantageux d'utiliser entre 0,005 % et 5 % en poids, de préférence entre 0,05 % et 0,5 % d'agent actif par rapport au poids total de la composition cosmétique finale.

Avantageusement, ledit agent actif est utilisé dans la composition en association avec au moins une substance choisie dans le groupe constitué par les vitamines, en particulier les vitamines du groupe A (rétinol), C et D3 et leurs dérivés tels que les esters notamment les palmitates et propionates, les tocophérols, les xanthines, en particulier la caféine ou la théophylline, les rétinoides, en particulier la vitamine A acide, les extraits de Centella asiatica, les acides asiatiques, madécassiques et leurs dérivés glycosylés tels que l'asiaticoside ou le madécassoside, les extraits de Siegesbeckia orientalis, les extraits de Commiphora mukul et les extraits d'Eriobotrya japonica, les dérivés de silicium cosmétiquement acceptables tels que des polysiloxanes, des silanols et des silicones, les acides aminés et leurs sels notamment de magnésium ou de calcium, en particulier l'acide arspartique, l'arginine, la citrulline et la thréonine, les céramides, les glycocéramides, les dérivés de sphingosine, en particulier les céramides de type II et III, les phospholipides, la forskoline et ses dérivés, les extraits de Coleus, les extraits de Tephrosia, les inhibiteurs d'élastase en particulier l'acide ellagique, les peptides de soja, les inhibiteurs de collagénase en particulier les peptides et les extraits végétaux tels que les extraits de racine de Coptidis, les extraits de racine de Scutellaria baicalensis Georgi, les flavonoïdes tels que la wogonine, la baicaline et la baicaléine, les extraits hydro-éthanoliques de feuilles de Ginkgo biloba, de Mosla chinensis, de Salvia officinalis, de Cinnamomum cassia, les extraits cathéchiques de Camellia sinensis et les extraits aqueux de coques de fèves de Theobroma cacao, les extraits de Phellodendron amurense, les anti-inflammatoires en particulier les inhibiteurs de phospholipase A2, les apaisants en particulier les extraits de réglisse, l'acide glycyrrhétinique, le glycyrrhizinate d'ammonium, les agents hydratants en particulier les polyols, le propylène glycol, le butylène glycol, le glycérol, l'acide hyaluronique, les agents anti-vergetures, en particulier les extraits de Marron d'Inde et l'escine, les agents protégeant ou améliorant la microcirculation, en particulier les bioflavonoïdes de Ginkgo biloba, l'isodon, les extraits d'Ami visnaga, la visnadine, la ruscogénine, les antiradicalaires en particulier les polyphénols tels que les OPC (Oligomères Procyanidoliques) et leurs dérivés, des extraits végétaux en particulier des extraits de Curcuma longa, les agents anti-séborrhéiques tels qu'un inhibiteur de 5-alpha-réductase, en particulier un extrait de Pygeum africanum, et les agents stimulant la microcirculation sanguine, tels que la cépharanthine et le nicotinate de méthyle, les saponines de luzerne, les sojasapogénols, en particulier de type A et de type B, les agents stimulants la synthèse du collagène VII tels que les extraits de Bertholletia, l'acide ellagique, le D-xylose et des complexes d'oligo-éléments.

Avantageusement, ledit agent actif est utilisé dans la composition en association avec au moins une substance choisie parmi les substances protégeant la peau des effets nocifs du soleil telles que les filtres solaires seuls ou en combinaison, notamment les filtres UV A et les filtres UV B, en particulier les oxydes de titane et les oxydes de zinc, l'oxybenzone, les cinnamates, en particulier l'octylméthoxycinnamate (commercialisé sous la marque Parsol MCX), le butylméthoxydibenzoylméthane (commercialisé sous la marque Parsol 1789) et les filtres d'origine végétale, les substances limitant les dommages causés à l'ADN, en particulier celles limitant la formation de dimères de thymine tel que l'acide ascorbique et ses dérivés et/ou le Photonyl, et les substances contribuant à l'élimination des taches de vieillissement tels que les inhibiteurs de la synthèse de mélanine ou de tyrosinase.

Selon une autre de ses caractéristiques essentielles, l'invention concerne une méthode de soin cosmétique. Cette méthode est plus particulièrement destinée à corriger une déficience de l'expression et/ou de la fonctionnalité des récepteurs membranaires CD44 des cellules de la peau.

Plus précisément, la méthode de soin cosmétique selon l'invention comprend l'application sur les zones de la peau à traiter d'une composition cosmétique contenant, à titre de principe actif, au moins un agent augmentant l'expression des récepteurs membranaires CD44 des cellules de la peau et/ou améliorant la fixation à la surface desdites cellules de la peau de l'acide hyaluronique, et/ou du collagène, en particulier du collagène I et/ou du collagène IV, et/ou de la fibronectine.

Selon la méthode de l'invention, le principe actif est constitué du gluconate de calcium ou d'un sel de l'acide gluconique associé à un sel de calcium.

Dans ce procédé cosmétique, on applique sur la partie de la peau à traiter une composition cosmétique contenant l'un des agents actifs tels que précédemment définis.

Cette méthode de soin cosmétique permet notamment de corriger la déficience de la fixation à la surface des cellules de la peau de l'acide hyaluronique et/ou du collagène, en particulier du collagène I et/ou du collagène IV, et/ou de la fibronectine.

Cette méthode de soin cosmétique permet notamment d'améliorer l'hydratation de l'épiderme.

L'invention concerne également une méthode de soin cosmétique telle que définie précédemment, destinée à corriger les effets négatifs du vieillissement sur l'expression et/ou sur la fonctionnalité des récepteurs membranaires CD44 des cellules de la peau. Ce point est particulièrement important puisqu'il a été démontré par les inventeurs de la présente invention que la capacité des kératinocytes à fixer l'acide hyaluronique diminuait considérablement au cours du vieillissement, traduisant ainsi une perte de la fonctionnalité du CD44.

Les exemples qui suivent sont donnés à titre purement illustratifs de la présente invention.

### Exemple 1

### Protocole du test permettant de mettre en évidence par fluorescence la fixation de l'acide hyaluronique à la surface des kératinocytes

### I - Mesure de la quantité d'acide hyaluronique fixé à la surface des kératinocytes

On réalise les étapes suivantes :

### 1 - Ensemencement des cellules:

15 000 kératinocytes humains sont ensemencés dans des puits de culture (plaques à 96 puits) dans un milieu K-SFM (Gibco).

### 2 - Obtention de la confluence des cultures :

Incubation des cultures pendant 72 h à 37°C sous atmosphère saturée en humidité, avec 5 % de CO₂ avec un renouvellement du milieu de culture après 48 h.

### 3 - Fixation d'acide hyaluronique :

Incubation des cellules pendant 4 h à 4°C avec de l'acide hyaluronique lié de façon covalente à un fluorochrome, la fluorescamine (HA-FA), synthétisée au laboratoire selon la technique décrite dans « Preparation and properties of fluoresceine-labelled hyaluronate ; Carbohydrat Research. 1975. Vol. 44. Pp 251-257 », préparé dans du K-SFM contenant 0,2 % d'azide de sodium.

### 4 - Evaluation de l'activité des produits testés :

Les produits pour lesquels on veut évaluer l'action sur la quantité de HA-FA fixée par les kératinocytes sont ajoutés dans le milieu d'incubation au début de l'incubation. Des contrôles sont effectués en absence des produits à évaluer et en présence de leur excipient ayant servi à les solubiliser.

### 5 - Fixation non spécifique de HA-FA :

Afin de déterminer le marquage non spécifique, on utilise rigoureusement les mêmes conditions expérimentales que lors de l'étape 3 mais en ajoutant 200 fois plus d'acide hyaluronique non marqué par un fluorochrome.

### 6 - Quantification de la fluorescence :

Après avoir retiré le milieu de fixation d'HA-FA, on effectue deux rinçages au PBS après quoi la fluorescence fixée sur les kératinocytes est mesurée à une longueur d'onde d'excitation λex = 485 nm et une longueur d'onde d'émission λem = 538 nm.

### 7 - Expression des résultats :

Les résultats sont exprimés en unité de fluorescence (fluorescence spécifique), déduction faite de la fluorescence non spécifique (voir 5-).

### 8 - Normalisation :

Dans certains cas, en particulier lorsque l'on veut comparer deux souches de cellules différentes, par exemple provenant de donneurs d'âges différents, ou ayant reçu un traitement différent, il est nécessaire de normaliser en tenant compte du nombre de cellules présentes dans les puits de culture. Pour cela, on ramène les valeurs de fluorescence mesurées à la teneur en ADN cellulaire effectué selon la méthode du DAPI (voir III-).

### II - Mesure de l'expression du récepteur de l'acide hyaluronique (CD44) à la surface des kératinocytes

Les étapes 1 et 2 sont identiques aux étapes 1 et 2 du I.

### 3 - Fixation des cellules au paraformaldéhyde à 2 % dans du PBS contenant du calcium et du magnésium pendant 5 min à température ambiante.

### 4 - Saturation des sites de liaison anticorps antigène non spécifiques :

Après deux rinçages au PBS, les cellules sont incubées avec du milieu de saturation PBS avec calcium et magnésium additionné de 5 % v/v de sérum de veau et de 0,05 % p/v d'azide de sodium pendant 1 h à température ambiante.

### 5 - Marquage :

Ajout de l'anticorps monoclonal IgG1 anti CD44 humain, couplé à la R-phycoérythrine dilué dans le milieu de saturation (voir 4-) au 1/50^{ème} pendant 1 h à température ambiante et à l'abri de la lumière.

6 - Un contrôle isotypique avec une IgG1 couplée à la R-phycoérythrine est réalisé dans les mêmes conditions expérimentales que pour le marquage. Les valeurs obtenues sont soustraites de celles du marquage.

### 7 - Quantification de la fluorescence :

Après 2 rinçages avec le milieu de saturation, la lecture de la fluorescence présente dans les puits est effectuée avec 50 µl de PBS à λ exitation = 544 nm et λ émission = 590 nm.

### 8 - Evaluation des produits à tester :

Les produits dont on veut évaluer l'activité sur l'expression du récepteur CD44 sont ajoutés dans le milieu de culture 24 h après l'ensemencement des cellules. Pour ce faire, le milieu d'ensemencement est retiré et remplacé par un milieu identique contenant la substance à tester. L'incubation se poursuit alors pendant 48 h. Des contrôles sont effectués en absence des produits à évalue, et en présence de l'excipient ayant servis à les solubiliser. Toutes choses égales par ailleurs.

### 9 - Mesure de la fonctionnalité du récepteur CD44 :

Après traitement des cultures pendant 48 h avec les substances à évaluer (voir 8-), une mesure de la capacité des cellules à fixer l'acide hyaluronique est réalisée selon le protocole I-, en ajoutant à l'étape 3 de ce protocole, du CaCl₂ à la concentration de 20 mM.

### 10 - Normalisation :

Les valeurs ainsi obtenues sont normalisées en tenant compte de la quantité de matériel cellulaire présente dans les puits. Pour cela, une mesure de la teneur en ADN cellulaire est effectuée par la méthode au DAPI (voir protocole ci-dessous).

### III - Quantification de l'ADN cellulaire par le dihydrochlorure de 4'6'-diaminephénylindol (DAPI)

La quantification de l'ADN cellulaire peut s'effectuer directement sur les cultures après quantification de l'expression du CD44.

### 1 - Etape de perméabilisation :

Dans chaque puits, on ajoute 30 µl de méthanol à -20°C pendant 1 min.

### 2 - Marquage au DAPI :

Après deux rinçages au PBS, on ajoute une solution de DAPI à 20 µg/ml pendant 15 min. à température ambiante et à l'obscurité.

### 3 - Quantification de la fluorescence :

Après 2 rinçages avec du PBS, la lecture de la fluorescence présente dans les puits est effectuée avec 50 µl de PBS à λ exitation = 355 nm et λ émission = 460 nm.

### Exemple 2

### Mise en évidence de l'effet du vieillissement sur la fonctionnalité du récepteur CD44 en ce qu'il fixe l'acide hyaluronique

Cette étude a été réalisée en utilisant le test dont le protocole (I-) est donné à l'exemple 1.

L'étude a été réalisée sur des cultures de kératinocytes humains normaux provenant de donneurs d'âges différents.

Les résultats sont donnés dans le tableau I ci-dessous.

**TABLEAU I**

| Ages des donneurs en années | Fixation d'acide hyaluronique (λem = 538 nm) rapportée à la quantité d'ADN (λem = 460 nm) | |
|---|---|---|
| | Moyenne | Ecart-type |
| 18 | 257 | 13 |
| 20 | 505 | 42 |
| 33 | 378 | 52 |
| 44 | 75 | 21 |
| 50 | 77 | 2 |
| 67 | 48 | 7 |

On observe une très forte diminution de la capacité des kératinocytes à fixer l'acide hyaluronique au cours du vieillissement traduisant une perte de la fonctionnalité du CD44.

### Exemple 3

### Effet de l'association du gluconate et du calcium sur la fixation de l'acide hyaluronique sur les kératinocytes

Les études qui suivent ont été réalisées selon le protocole I- de l'exemple 1.

a) Dans une première expérience, on a comparé la fixation d'acide hyaluronique marqué par un fluorochrome sur des kératinocytes humains en culture en présence de CaCl₂ et de gluconate de calcium à molarité égale.

Les résultats sont donnés dans le tableau II ci-dessous.

**TABLEAU II**

| Concentrations | Fluorescence mesurée (UF) | | | |
|---|---|---|---|---|
| | CaCl₂ | | Gluconate de calcium | |
| en mM | Moyenne | écart type | Moyenne | écart type |
| 5 | 30 | 9 | 310* | 30 |
| 10 | 370 | 21 | 2 745* | 125 |

| | | | | |
|---|---|---|---|---|
| * Différences hautement significatives entre CaCl₂ et gluconate de Calcium. | | | | |

On observe qu'en présence de gluconate de calcium, la quantité de fluorescence mesurée est significativement beaucoup plus forte que pour le chlorure de calcium à molarité équivalente. Cela indique que des quantités beaucoup plus fortes d'acide hyaluronique se sont fixées sur les cultures de kératinocytes en présence de ce produit qu'en présence de chlorure de calcium.

b) Dans une deuxième expérience, on a comparé l'action de concentrations croissantes de CaCl₂ en présence ou en absence de gluconate de sodium à 10 mM.

Les résultats figurent dans le tableau III ci-dessous.

**TABLEAU III**

| Concentrations CaCl₂ en mM | Fluorescence mesurée (UF) | | | |
|---|---|---|---|---|
| | sans gluconate de Na | | avec gluconate de Na (10 mM) | |
| | Moyenne | écart type | Moyenne | écart type |
| 0 | 6 | 11 | 6 | 11 |
| 2,5 | 8 | 15 | 27 | 36 |
| 5 | 7 | 16 | 84* | 41 |
| 10 | 447 | 43 | 2 186* | 350 |

| | | | | |
|---|---|---|---|---|
| * Différences hautement significatives entre CaCl₂ seul et en présence de gluconate de sodium. | | | | |

Ce résultat confirme l'effet positif du calcium sur la fixation d'acide hyaluronique. L'effet positif du calcium se manifeste à la concentration de 10 mM.

En présence de gluconate sous la forme de gluconate de sodium, la quantité d'acide hyaluronique fixée avec 10 mM de CaCl₂ est plus forte. Par ailleurs, à la concentration de CaCl₂ de 5 mM, sans effet sur cette fixation, l'ajout de gluconate de Na permet d'observer une fixation significative d'acide hyaluronique.

c) Dans une troisième expérience, on a fixé la concentration de calcium à 10 mM et fait varier la concentration de gluconate apporté sous forme de sel de sodium.

Les résultats sont consignés dans le tableau IV ci-dessous.

**TABLEAU IV**

| Gluconate de Na Concentration en mM | Fluorescence mesurée (UF) en présence de 10 mM de CaCl₂ | |
|---|---|---|
| | Moyenne | Ecart-type |
| 0 | 447 | 43 |
| 2,5 | 5 241* | 597 |
| 5 | 4 535* | 616 |
| 10 | 2 186* | 350 |

| | | |
|---|---|---|
| * Différences hautement significatives entre CaCl₂ seul et en présence de gluconate de sodium. | | |

On voit ainsi clairement que le gluconate apporté sous forme de gluconate de sodium induit de manière dose-dépendante une potentialisation de l'effet du calcium et permet ainsi la fixation de quantité d'acide hyaluronique beaucoup plus importante qu'en présence de calcium uniquement. Cette expérience montre qu'il existe des rapports molaires Ca/Gluconate préférentiels proches de 4 (10 mM/2,5 mM).

Cette dernière expérience est utile pour ajuster la quantité de gluconate à celle du calcium pour produire le maximum d'action.

On voit que dans des conditions relativement optimisées, le gluconate de sodium permet au moins d'augmenter les effets du calcium d'un facteur 12, ce qui est considérable.

Ces deux derniers éléments ont de l'importance dans la mesure où le gluconate de sodium peut potentialiser l'effet du calcium, présent en abondance dans l'épiderme, sur la fixation de l'acide hyaluronique à la surface des kératinocytes dans l'épiderme.

On voit donc que l'invention ne se limite pas seulement au gluconate de Ca mais concerne plus généralement le gluconate sous la forme de sel ou de complexe de calcium, de magnésium, de manganèse, de zinc ou de sodium ou de tout autre cation divalent ou monovalent.

### Exemples de formulations cosmétiques utilisant les propriétés des agents stimulant la fixation de l'acide hyaluronique sur les cellules cutanées

| Exemple 4 : Emulsion hydratante et nourrissante anti-âge | |
|---|---|
| Gluconate de calcium | 0,1 g |
| Palmitate de vitamine A | 0,01 g |
| Vitamine E phosphate | 0,01 g |
| Vitamine C magnésium phosphate | 0,2 g |
| Céramides de blé | 0,2 g |
| Protéines de blé | 1 g |
| Filtre UVA-UVB | 5 g |
| Excipient avec conservateurs et parfums | qsp 100 g |

Cette émulsion nourrissante améliore l'hydratation cutanée en particulier de l'épiderme, la finesse et le grain de la peau et atténue les rides. Elle est appliquée quotidiennement sur le visage.

| Exemple 5 : Gel hydratant raffermissant et restructurant | |
|---|---|
| Gluconate de calcium | 0,1 g |
| Aspartate de magnésium | 0,1 g |
| Extrait titré de Centella asiatica | 0,1 g |
| Saponines de soja | 0,05 g |
| Vitamine C magnésium phosphate | 0,1 g |
| Excipient avec conservateurs et parfums | qsq 100 g |

Ce gel hydratant exerce un effet tenseur sur la peau et en améliore la souplesse et la fermeté. Elle est appliquée quotidiennement sur le visage, le cou, le buste.

| Exemple 6 : Lotion hydratante et tonifiante | |
|---|---|
| Extrait de Panax Ginseng | 0,2 g |
| AMP cyclique | 0,05 g |
| Caféine | 0,1 g |
| Excipient avec conservateurs et parfums | qsp 100 g |

Cette lotion hydratante est utilisée en application quotidienne sur le visage, de préférence le matin, pour obtenir une peau plus belle et plus éclatante.

| Exemple 7 : Mascara hydratant | |
|---|---|
| Acide hyaluronique | 0,5 g |
| D-xylose | 0,3 g |
| Pigments colorés | 10 g |
| Cires | 30 g |
| Excipient | qsp 100 g |

## Revendications

1. Utilisation non thérapeutique dans une composition cosmétique, du gluconate de calcium ou d'un sel de l'acide gluconique associé à un sel de calcium, en tant qu'agent actif améliorant l'hydratation de l'épiderme et/ou du derme ou améliorant la fixation à la surface des cellules de la peau de l'acide hyaluronique, et/ou du collagène, en particulier du collagène I et/ou du collagène IV, et/ou de la fibronectine.

2. Utilisation selon la revendication 1, **caractérisée en ce que** ledit agent actif augmente l'expression des récepteurs CD44 des kératinocytes et/ou des fibroblastes et/ou améliore la fixation de l'acide hyaluronique à la surface desdites cellules.

3. Utilisation selon la revendication 1, **caractérisée en ce que** ledit agent actif augmente l'expression des récepteurs CD44 des fibroblastes et/ou améliore la fixation à la surface desdits fibroblastes du collagène, en particulier du collagène I et/ou du collagène IV, et/ou de la fibronectine.

4. Utilisation selon l'une des revendications 1 à 3, **caractérisée en ce que** ledit agent actif est destiné à améliorer l'hydratation de l'épiderme.

5. Utilisation selon l'une des revendications 1 à 4, **caractérisée en ce que** ledit agent actif est destiné à corriger les effets négatifs du vieillissement sur l'expression et/ou la fonctionnalité des récepteurs CD44 des cellules de la peau.

6. Utilisation selon l'une des revendications 1 à 5, **caractérisée en ce que** ladite composition est une composition destinée à hydrater et raffermir la peau.

7. Utilisation selon l'une des revendications 1 à 5, **caractérisée en ce que** ladite composition est une composition destinée à hydrater et restructurer la peau.

8. Utilisation selon l'une des revendications 1 à 7, **caractérisée en ce que** ledit agent actif est le gluconate de calcium.

9. Utilisation selon l'une des revendications 1 à 7, **caractérisée en ce que** ledit agent actif est une association d'un sel d'acide gluconique et d'un sel de calcium.

10. Utilisation selon la revendication 9, **caractérisée en ce que** ledit sel de calcium est le chlorure de calcium.

11. Utilisation selon l'une des revendications 9 ou 10, **caractérisée en ce que** ledit sel d'acide gluconique est choisi dans le groupe constitué par les sels de sodium, de calcium, de magnésium, de zinc et de manganèse.

12. Utilisation selon l'une des revendications 9 à 11, **caractérisée en ce que** le rapport molaire calcium/gluconate est compris entre 2 et 6.

13. Utilisation selon la revendication 12, **caractérisée en ce que** ledit rapport est de l'ordre de 4.

14. Utilisation selon l'une des revendications 1 à 13, **caractérisée en ce que** la proportion dudit agent actif dans la composition est comprise entre 0,005% et 5% en poids, de préférence entre 0,05% et 0,5%, par rapport au poids total de la composition cosmétique finale.

15. Utilisation selon l'une des revendications 1 à 14, **caractérisée en ce que** ledit agent actif est utilisé dans la composition en association avec au moins une substance choisie dans le groupe constitué par les vitamines, en particulier les vitamines du groupe A (rétinol), C et D3 et leurs dérivés tels que les esters notamment les palinitates et propionates, les tocophérols, les xanthines, en particulier la caféine ou la théophylline, les rétinoides, en particulier la vitamine A acide, les extraits de Centella asiatica, les acides asiatiques, madécassiques et leurs dérivés glycosylés tels que l'asiaticoside ou le madécassoside, les extraits de Siegesbeckia orientalis, les extraits de Commiphora mukul et les extraits d'Eriobotrya japonica, les dérivés de silicium cosmétiquement acceptables tels que des polysiloxanes, des silanols et des silicones, les acides aminés et leurs sels notamment de magnésium ou de calcium, en particulier l'acide arspartique, l'arginine, la citrulline et la thréonine, les céramides, les glycocéramides, les dérivés de sphingosine, en particulier les céramides de type II et III, les phospholipides, la forskoline et ses dérivés, les extraits de Coleus, les extraits de Tephrosia, les inhibiteurs d'élastase en particulier l'acide ellagique, les peptides de soja, les inhibiteurs de collagénase en particulier les peptides et les extraits végétaux tels que les extraits de racine de Coptidis, les extraits de racine de Scutellaria baicalensis Georgi, les flavonoïdes tels que la wogonine, la baicaline et la baicaléine, les extraits hydro-éthanoliques de feuilles de Ginkgo biloba, de Mosla chinensis, de Salvia officinalis, de Cinnamomum cassia, les extraits cathéchiques de Camellia sinensis et les extraits aqueux de coques de fèves de Theobroma cacao, les extraits de Phellodendron amurense, les anti-inflammatoires en particulier les inhibiteurs de phospholipase A2, les apaisants en particulier les extraits de réglisse, l'acide glycyrrhétinique, le glycyrrhizinate d'ammonium, les agents hydratants en particulier les polyols, le propylène glycol, le butylène glycol, le glycérol, l'acide hyaluronique, les agents anti-vergetures, en particulier les extraits de Marron d'Inde et l'escine, les agents protégeant ou améliorant la microcirculation, en particulier les bioflavonoïdes de Ginkgo biloba, l'isodon, les extraits d'Ami visnaga, la visnadine, la ruscogénine, les antiradicalaires en particulier les polyphénols tels que les OPC (Oligomères Procyanidoliques) et leurs dérivés, des extraits végétaux en particulier des extraits de Curcuma longa, les agents anti-séborrhéiques tels qu'un inhibiteur de 5-alpha-réductase, en particulier un extrait de Pygeum africanum, et les agents stimulant la microcirculation sanguine, tels que la cépharanthine et le nicotinate de méthyle, les saponines de luzerne, les sojasapogénols, en particulier de type A et de type B, les agents stimulants la synthèse du collagène VII tels que les extraits de Bertholletia, l'acide ellagique, le D-xylose et des complexes d'oligo-éléments.

16. Utilisation selon l'une des revendications 1 à 15, **caractérisée en ce que** ledit agent actif est utilisé dans la composition en association avec au moins une substance choisie parmi les substances protégeant la peau des effets nocifs du soleil telles que les filtres solaires seuls ou en combinaison, notamment les filtres UV A et les filtres UV B, en particulier les oxydes de titane et les oxydes de zinc, l'oxybenzone, les cinnamates, en particulier l'octylméthoxycinnamate, le butylinéthoxydibenzoylméthane et les filtres d'origine végétale, les substances limitant les dommages causés à l'ADN, en particulier celles limitant la formation de dimères de thymine tel que l'acide ascorbique et ses dérivés et/ou le Photonyl, et les substances contribuant à l'élimination des taches de vieillissement tels que les inhibiteurs de la synthèse de mélanine ou de tyrosinase.

## Patentansprüche

1. Nichttherapeutische Verwendung einer kosmetischen Zusammensetzung von Calciumgluconat oder einem Gluconsäuresalz in Verbindung mit einem Calciumsalz als Wirkstoff, der die Hydratation der Epidermis und/oder Dermis verbessert oder die Fixierung an der Oberfläche der Hautzellen von Hyaluronsäure und/oder Collagen verbessert, insbesondere des Collagens I und/oder des Collagens IV und/oder des Fibronectins.

2. Verwendung nach Anspruch 1, **dadurch gekennzeichnet, dass** der Wirkstoff die Expression der CD44-Rezeptoren der Kreatinocyten und/oder der Fibroblasten verstärkt und/oder die Fixierung der Hyaluronsäure an der Oberfläche dieser Zellen verbessert.

3. Verwendung nach Anspruch 1, **dadurch gekennzeichnet, dass** der Wirkstoff die Expression der CD44-Rezeptoren der Fibroblasten verbessert und/oder die Fixierung an der Oberfläche dieser Fibroblasten des Collagens verbessert, insbesondere des Collagens I und/oder des Collagens IV und/oder des Fibronectins.

4. Verwendung nach einen der Ansprüche 1 bis 3, **dadurch gekennzeichnet, dass** der Wirkstoff dazu vorgesehen ist, die Hydratation der Epidermis zu verbessern.

5. Verwendung nach einem der Ansprüche 1 bis 4, **dadurch gekennzeichnet, dass** der Wirkstoff dazu vorgesehen ist, die negativen Wirkungen des Alterns auf die Expression und/oder die Funktionalität der CD44-Rezeptoren der Hautzellen zu korrigieren.

6. Verwendung nach einem der Ansprüche 1 bis 5, **dadurch gekennzeichnet, dass** die Zusammensetzung eine Zusammensetzung ist, die dazu vorgesehen ist, die Haut zu hydratisieren und zu straffen.

7. Verwendung nach einem der Ansprüche 1 bis 5, **dadurch gekennzeichnet, dass** die Zusammensetzung eine Zusammensetzung ist, die dazu vorgesehen ist, die Haut zu hydratisieren und zu restrukturieren.

8. Verwendung nach einem der Ansprüche 1 bis 7, **dadurch gekennzeichnet, dass** der Wirkstoff Calciumgluconat ist.

9. Verwendung nach einem der Ansprüche 1 bis 7, **dadurch gekennzeichnet, dass** der Wirkstoff eine Zuordnung eines Gluconsäuresalzes und eines Calciumsalzes ist.

10. Verwendung nach Anspruch 9, **dadurch gekennzeichnet, dass** das Calciumsalz Calciumchlorid ist.

11. Verwendung nach einem der Ansprüche 9 oder 10, **dadurch gekennzeichnet, dass** das Gluconsäuresalz gewählt wird aus der Gruppe, die besteht aus den Salzen von Natrium, Calcium, Magnesium, Zink und Mangan.

12. Verwendung nach einem der Ansprüche 9 bis 11, **dadurch gekennzeichnet, dass** das Molverhältnis Calcium/Gluconat zwischen 2 und 6 liegt.

13. Verwendung nach Anspruch 12, **dadurch gekennzeichnet, dass** das Verhältnis in der Größenordnung von 4 liegt.

14. Verwendung nach einem der Ansprüche 1 bis 13, **dadurch gekennzeichnet, dass** der Anteil des Wirkstoffs in der Zusammensetzung zwischen 0,005 Gew.% und 5 Gew.-%, vorzugsweise zwischen 0,05 Gew.-% und 0,5 Gew.-% im Verhältnis zum Gesamtgewicht der endgültigen kosmetischen Zusammensetzung liegt.

15. Verwendung nach einem der Ansprüche 1 bis 14, **dadurch gekennzeichnet, dass** der Wirkstoff in der Zusammensetzung in Verbindung mit wenigstens einer Substanz verwendet wird, die gewählt ist aus der Gruppe, die besteht aus den Vitaminen, insbesondere den Vitaminen der Gruppe A (Retinol), C und D3 und deren Derivaten wie Estern, insbesondere den Palmitaten und Propionaten, den Tocopherolen, den Xanthinen und insbesondere Koffein oder Theophyllin, den Retinoiden, insbesondere Vitamin-A-Säure, den Extrakten von Centella asiatica, den Asiatsäuren, madecassischen Säuren und deren glykosylierten Derivaten wie Asiaticosid oder Madekassosid, den Extrakten von Siegesbeckia orientalis, den Extrakten von Commiphora mukul und den Extrakten von Eriobotyra japonica, den kosmetisch verträglichen Siliciumderivaten wie Polysiloxanen, Silanolen und Siliconen, den aminierten Säuren und deren Salzen, insbesondere von Magnesium oder von Calcium, insbesondere Aspartatsäure, Arginin, Citrullin und Threonin, den Ceramiden, den Glycoceramiden, den Sphingosinderivaten, insbesondere den Ceramiden vom Typ II und III, den Phospholipiden, Forskolin und seinen Derivaten, den Coleus-Extrakten, den Tephrosia-Extrakten, den Elastaseinhibitoren, insbesondere Ellaginsäure, den Sojapeptiden, den Collagenaseinhibitoren, insbesondere den Peptiden und den pflanzlichen Extrakten wie Wurzelextrakten von Coptidis, den Wurzelextrakten von Scutellaria baicalensis Georgi, den Flavonoiden wie Wogonin, Baicalin und Baicalein, den hydroethanolischen Blattextrakten von Ginkgo biloba, Mosla chinensis, Salvia officinalis, Cassiazimt, den kathecholischen Extrakten von Camellia sinensis und den wässrigen Kokosfaserextrakten von Theobroma cacao, den Extrakten von Phellodendron amurense, den Antientzündungsmitteln, insbesondere die Inhibitoren von Phospholipase A2, den Beruhigungsmitteln, insbesondere den Extrakten von Süßholz, Glycyrrhetinsäure, Ammonium-Glycyrrhizinat, den hydratisierenden Mitteln, insbesondere Polyolen, Propylenglykol, Butylenglykol, Glycerol, Hydraluronsäure, den Antifaltenmitteln, insbesondere den Rosskastanienextrakten und Escin, den die Mikrozirkulation schützenden oder verbessernden Mitteln, insbesondere den Bioflavonoiden von Ginkgo biloba, Isodon, den Extrakten von Ammi visnaga, Visnadin, Ruscogenin, den Antiradikalmitteln, insbesondere den Polyphenolen wie OPC (Procyanidololigomere) und deren Derivaten, den Pflanzenextrakten, insbesondere den Extrakten von Curcuma longa, den Antipickelmitteln wie einem Inhibitor von 5-Alpha-Reduktase, insbesondere einem Extrakt von Pygeum africanum und dem die Blutmikrozirkulation stimulierenden Mitteln wie Cepharantin und Methylnikotinat, Luzernen-Saponinen, Sojasapogenolen, insbesondere vom Typ A und Typ B, den die Synthese von Collagen VII stimulierenden Mitteln wie die Extrakte von Bertholletia, Ellaginsäure, D-Xylose und den Komplexen von Oligoelementen.

16. Verwendung nach einem der Ansprüche 1 bis 15, **dadurch gekennzeichnet, dass** der Wirkstoff in der Zusammensetzung in Verbindung mit wenigstens einer Substanz verwendet wird, die gewillt ist unter den die Haut vor schädlichen Wirkungen der Sonne schützenden Substanzen wie Sonnenfiltern allein oder in Kombination, insbesondere die Filter UV A und die Filter UV B, insbesondere die Titanoxide und Zinkoxide, Oxybenzon, die Cinnamate, insbesondere Octylmethoxycinnamat, Butylmethoxydibenzoylmethan und die Filter pflanzlichen Ursprungs, die Substanzen, die die an der DNA hervorgerufenen Schäden begrenzen, insbesondere jene, die die Bildung von Dimeren von Thymin begrenzen wie Ascorbinsäure und ihre Derivate und/oder Photonyl und die Substanzen, die zur Entfernung von Alterungsfalten beitragen wie die Inhibitoren der Synthese von Melanin oder von Tyrosinase.

## Claims

1. Non-therapeutic use in a cosmetic composition, of calcium gluconate or of a salt of gluconic acid combined with a calcium salt, as an active agent improving moisturization of the epidermis and/or of the dermis or improving the binding to the surface of the skin cells of hyaluronic acid, and/or of collagen, in particular of collagen I and/or collagen IV, and/or of fibronectin.

2. Use according to Claim 1, **characterized in that** said active agent increases the expression of the CD44 receptors of keratinocytes and/or of fibroblasts and/or improves the binding of hyaluronic acid to the surface of said cells.

3. Use according to Claim 1, **characterized in that** said active agent increases the expression of the CD44 receptors of fibroblasts and/or improves the binding to the surface of said fibroblasts, of collagen, in particular of collagen I and/or collagen IV, and/or of fibronectin.

4. Use according to one of Claims 1 to 3, **characterized in that** said active agent is intended to improve moisturization of the epidermis.

5. Use according to one of Claims 1 to 4, **characterized in that** said active agent is intended to correct the negative effects of ageing on the expression and/or the functionality of the CD44 membrane receptors of skin cells.

6. Use according to one of Claims 1 to 5, **characterized in that** said composition is a composition intended to moisturize and firm the skin.

7. Use according to one of Claims 1 to 5, **characterized in that** said composition is a composition intended to moisturize and restructure the skin.

8. Use according to one of Claims 1 to 7, **characterized in that** said active agent is calcium gluconate.

9. Use according to one of Claims 1 to 7, **characterized in that** said said active agent is an association of a salt of gluconic acid and of a calcium salt.

10. Use according to Claim 9, **characterized in that** said calcium salt is calcium chloride.

11. Use according to one of Claims 9 or 10, **characterized in that** said salt of gluconic acid is selected from the group consisting of sodium, calcium, magnesium, zinc and manganese salts.

12. Use according to one of Claims 9 to 11, **characterized in that** the molar ratio of calcium to gluconate is between 2 and 6.

13. Use according to Claim 12, **characterized in that** said molar ratio is of the order of 4.

14. Use according to one of Claims 1 to 13, **characterized in that** the proportion of said active agent in the composition is between 0.005% and 5% by weight, preferably between 0.05% and 0.5%, by weight relative to the total weight of the final cosmetic composition.

15. Use according to one of Claims 1 to 14, **characterized in that** the said active agent is used in the composition in combination with at least one substance chosen from the group consisting of vitamins, in particular vitamins of group A (retinol), C and D3 and derivatives thereof such as esters, in particular the palmitates and propionates, tocopherols, xanthines, in particular caffeine or theophylline, retinoids, in particular vitamin A acid, extracts of Centella asiatica, asiatic acid and madecassic acid and glycosyl derivatives thereof, such as asiaticoside or madecassoside, extracts of Siegesbeckia orientalis, extracts of Commiphora mukul and extracts of Eriobotrya japonica, cosmetically acceptable silicon derivatives such as polysiloxanes, silanols and silicones, amino acids and salts thereof, especially the magnesium or calcium salts, in particular aspartic acid, arginine, citrulline and threonine, ceramides, glycoceramides, sphingosine derivatives, in particular ceramides of type II and type III, phospholipids, forskolin and derivatives thereof, extracts of Coleus, extracts of Tephrosia, elastase inhibitors, in particular ellagic acid, soybean peptides, collagenase inhibitors, in particular peptides and plant extracts such as extracts of Coptidis root, extracts of Scutellaria baicalensis Georgi root, flavonoids such as wogonine, baicaline and baicaleine, aqueous-ethanolic extracts of leaves of Ginkgo biloba, of Mosla chinensis, of Salvia officinalis, of Cinnamomum cassia, cathechic extracts of Camellia sinensis and aqueous extracts of Theobroma cacao bean husks, extracts of Phellodendron amurense, anti-inflammatory agents, in particular phospholipase A2 inhibitors, calmants, in particular extracts of liquorice, glycyrrhetinic acid, ammonium glycyrrhizinate, moisturizers, in particular polyols, propylene glycol, butylene glycol, glycerol, hyaluronic acid, anti-stretch mark agents, in particular extracts of common horse chestnut and escin, agents for protecting or improving the microcirculation, in particular bioflavonoids from Ginkgo biloba, isodon, extracts of Ami visnaga, visnadine, ruscogenin, free-radical scavengers, in particular polyphenols such as PCOs (Procyanidol Oligomers) and derivatives thereof, plant extracts, in particular extracts of Curcuma longa, anti-seborrhoeic agents such as a 5-α-reductase inhibitor, in particular an extract of Pygeum africanum, and agents for stimulating the blood microcirculation, such as cepharanthine and methyl nicotinate, alfalfa saponins, soya sapogenols, in particular of type A and type B, agents for stimulating collagen VII synthesis, such as extracts of Bertholletia, ellagic acid, D-xylose and trace element complexes.

16. Use according to one of Claims 1 to 15, **characterized in that** said active agent is used in the composition in combination with at least one substance chosen from substances for protecting the skin against the harmful effects of sunlight, such as sunscreens alone or in combination, in particular UVA screening agents and UVB screening agents, in particular titanium oxides and zinc oxides, oxybenzone, cinnamates, in particular octyl methoxycinnamate, butylmethoxydibenzoylmethane and screening agents of plant origin, substances for limiting the damage caused by DNA, in particular those limiting the formation of thymine dimers, such as ascorbic acid and derivatives thereof and/or Photonyl, and substances for contributing towards removing age marks, such as inhibitors of melanin synthesis or tyrosinase inhibitors.
